(19) Europäisches Patentamt
European Patent Office
Office européen des brevets

(11) Publication number: 0 032 733
B1

(12) # EUROPEAN PATENT SPECIFICATION

(45) Date of publication of patent specification: 13.06.84

(51) Int. Cl.³: C 07 C 125/073, C 07 C 125/077

(21) Application number: 81100303.7

(22) Date of filing: 16.01.81

(54) **Process for preparing polymethylene polyphenyl polycarbamates.**

(30) Priority: 16.01.80 JP 2568/80

(43) Date of publication of application:
29.07.81 Bulletin 81/30

(45) Publication of the grant of the patent:
13.06.84 Bulletin 84/24

(84) Designated Contracting States:
DE FR GB NL

(56) References cited:
EP - A - 0 016 441
DE - C - 1 042 891
US - A - 4 146 727

(73) Proprietor: MITSUI TOATSU CHEMICALS, INCORPORATED
2-5 Kasumigaseki 3-chome
Chiyoda-Ku Tokyo 100 (JP)

(72) Inventor: Miyata, Katsuharu
1071-2, Nakanocho Totsuka-ku
Yokohama Kanagawa-ken (JP)
Inventor: Hasegawa, Seiji
1541, Yabecho Totsuka-ku
Yokohama Kanagawa-ken (JP)
Inventor: Aoki, Shinobu
1612, Kosugayacho Totsuka-ku
Yokohama Kanagawa-ken (JP)
Inventor: Hara, Isao
372-6, Ninomiya Ninomiyacho
Naka-gun Kanagawa-ken (JP)

(74) Representative: Zumstein, Fritz jun., Dr. et al,
Dr. F. Zumstein sen. Dr. E. Assmann Dr. R. Koenigsberger Dipl.-Ing. F. Klingseisen Dr. F. Zumstein jun. Bräuhausstrasse 4
D-8000 München 2 (DE)

EP 0 032 733 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Background of the Invention

1. *Field of the Invention*

This invention relates to a process for preparing diphenylmethane dicarbamates and polymethylene polyphenyl polycarbamates. More particularly, it relates to a process for preparing diphenylmethane dicarbamates and polymethylene polyphenyl polycarbamates for reacting an N-phenyl carbamic acid ester with formaldehyde or a formaldehyde-producing substance in the presence of a ferric halide compound and to such a process which permits reuse of the ferric halide compound.

Description of the Prior Art

Diphenylmethane dicarbamates and polymethylene polyphenyl polycarbamates are substances that are useful in the manufacture of agricultural chemicals, drugs, polyamides, polyurethanes and the like. In addition, diphenylmethane dicarbamates and polymethylene polyphenyl polycarbamates may be thermally decomposed to produce the corresponding diphenylmethane diisocyanates and polymethylene polyphenyl polyisocyanates. Accordingly, it is desired to develop new processes for preparing diphenylmethane dicarbamates and polymethylene polyphenyl polycarbamates with industrial advantages.

One well-known process for preparing diphenylmethane dicarbamates and polymethylene polyphenyl polycarbamates comprises reacting a corresponding isocyanate with an alcohol. However, this process involves the use of highly toxic aniline and phosgene in the preparation of the isocyanate starting material and, moreover, requires a complicated procedure.

Another well-known process for preparing diphenylmethane dicarbamates and polymethylene polyphenyl polycarbamates comprises reacting a corresponding amine compound with a chloroformic acid alkyl ester. In this process, however, the amine compound and chloroformic acid alkyl ester used as the starting materials both have such severe intoxicating and irritating properties that they are difficult to handle, and a complicated procedure is required. For these reasons, this process cannot be regarded as useful in industrial applications.

There is still another well-known process for preparing diphenylmethane dicarbamates and polymethylene polyphenyl polycarbamates by reacting an N-phenyl carbamic acid ester with formaldehyde. For example, as is described in German Patent No. 1,042,891 and U.S. Patent No. 2,946,768, N-phenyl ethyl carbamate and formaldehyde are heated in an aqueous solution of hydrochloric acid to obtain a condensation product thereof. However, this reaction is so slow that a large amount of unreacted N-phenyl carbamic acid ester remains even after the reaction has been carried out at high temperatures for a long period of time. Moreover, the resulting condensation product contains, in addition to the desired diphenylmethane dicarbamates and polymethylene polyphenyl polycarbamates, from 15 to 50% by weight of an [(alkoxycarbonyl) phenylaminomethyl] phenyl carbamic acid alkyl ester (hereinafter referred to as an N-benzyl compound) and various binuclear, trinuclear, tetranuclear and other polynuclear derivatives thereof, as well as a bis (N-carboalkoxyanilino)methane (hereinafter referred to as an anilinomethane compound) and the like. Most of these by-products are undesirable because they can hardly be converted into isocyanates by thermal decomposition. In order to solve this problem, U.S. Patent No. 4,146,727 has proposed a process in which the N-benzyl compounds formed as a by-product during the condensation reaction in a dilute aqueous solution of an acid are converted into diphenylmethane dicarbamates and polymethylene polyphenyl polycarbamates by heating the condensation product, together with a protonic acid or a Lewis acid, in a substantially anhydrous state. However, this process is a two-step one which comprises first carrying out a condensation reaction in a dilute aqueous solution of an acid to obtain a condensation product and then subjecting it to rearrangement in the presence of a different catalyst and under different reaction conditions. For this reason, it has the disadvantage of involving troublesome operations. Moreover, there still remains the problem that the first step (that is, the condensation reaction in a dilute aqueous solution of an acid) is low in reaction rate.

Summary of the Invention

It is an object of the present invention to provide a process for preparing polymethylene polyphenyl polycarbamates by reacting an N-phenyl carbamic acid ester with formaldehyde in which a ferric halide compound is used as the catalyst and the reaction is carried out in the presence of a solvent and in a substantially homogeneous system, whereby a higher reaction rate is achieved as compared with prior art processes, only a little or virtually no formation of by-products such as N-benzyl compound and the like is noted, and the desired product is obtained in good yield and with high selectivity.

It is another object of the present invention to provide a process for preparing polymethylene polyphenyl polycarbamates in which compositions consisting of polynuclear compounds in a proportion suitable for the manufacture of polyurethanes can be obtained by selecting appropriate reaction conditions and a proper reaction method.

It is still another object of the present invention to provide a process for preparing polymethylene

polyphenyl polycarbamates in which the ferric halide compound used as the catalyst can be easily and efficiently recovered by bringing it into contact with an aqueous solution of a hydrohalogenic acid and, moreover, the formation of undesirable by-products as described above can be substantially eliminated by repeated use of the ferric halide compound thus recovered.

According to the present invention, there is provided an improved process for preparing the polymethylene polyphenyl polycarbamate of the general formula

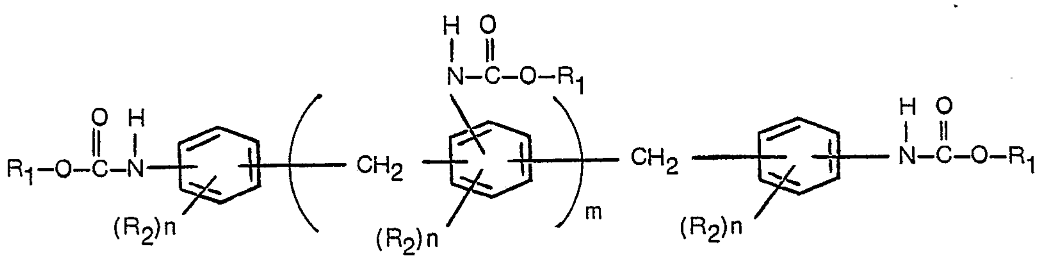 (I)

where $R_1$ is a lower alkyl radical of from 1 to 6 carbon atoms or a cycloalkyl radical, $R_2$ is a hydrogen atom, a halogen atom, a lower alkyl radical of from 1 to 6 carbon atoms, or a lower alkoxy radical of from 1 to 6 carbon atoms, n is a positive integer of from 1 to 3, and m is zero or a positive integer of from 1 to 5, by reacting an N-phenyl carbamic acid ester of the general formula

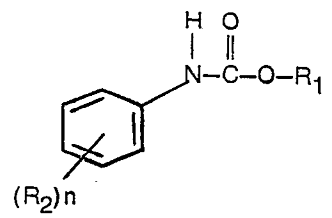 (II)

where $R_1$, $R_2$ and n have the same meanings as described above, with formaldehyde or a formaldehyde-producing substance, characterised by

(a) using a ferric halide compound as the catalyst in an amount of from 0.01 to 10 moles per mole of the N-phenyl carbamic acid ester and not less than 0.1% by weight based on the total amount of the reaction mixture, and

(b) carrying out the reaction in the presence of a solvent and in a substantially homogeneous system.

In addition, there is also provided an improved process for preparing polymethylene polyphenyl polycarbamates in which, after completion of the reaction, water or an aqueous solution of a hydrohalogenic acid is added to the reaction mixture and the resulting aqueous phase containing the ferric halide compound is separated and recovered to reuse the catalyst.

Prior to the present invention, no process has been known in which the ferric halide compound is used as the catalyst for the condensation reaction of an N-phenyl carbamic acid ester with formaldehyde. It is difficult from an industrial point of view to use generally known Lewis acids other than ferric halide compounds, because they have various disadvantages such as low activity, decomposition during the reaction and hence difficulty in recovery and reuse, and the like. In the aforementioned German Patent No. 1,042,891, for example, it is described that zinc chloride can be used as the catalyst. However, not only its catalytic activity is so low that large amounts of unreacted starting materials are recovered, but also very large amounts of N-benzyl compounds and anilinomethane compounds are formed as by-products. Aluminum chloride, tin chloride, nickel chloride, chromium chloride and the like are also low in activity. Boron trifluoride has high catalytic activity, but is expensive. In addition, it has the disadvantage that its recovery is low because of hydrolysis during the reaction and the hydrogen fluoride resulting from such hydrolysis brings about severe corrosion of the reactor. The process of the present invention overcomes the above-described difficulties and makes it possible to prepare polymethylene polyphenyl polycarbamates with great industrial advantages.

Detailed Description of the Invention

The N-phenyl carbamic acid ester used in the process of the present invention is a compound represented by the general formula (II). For example, useful N-phenyl carbamic acid esters are compounds of the general formula (II) in which $R_1$ is an alkyl radical such as methyl, ethyl, *n*-propyl, isopropyl, *n*-butyl, *sec*-butyl, isobutyl, *tert*-butyl, any of the pentyl radicals derived from *n*-pentane and its isomers, any of the hexyl radicals derived from *n*-hexane and its isomers, etc.; or a cycloalkyl radical such as cyclopentyl, cyclohexyl, etc.; and $R_2$ is a hydrogen atom; a halogen atom such as chlorine,

bromine, fluorine, etc.; an alkyl radical such as methyl, ethyl *n*-propyl, isopropyl, *n*-butyl, *sec*-butyl, isobutyl, *tert*-butyl, any of the pentyl radicals derived from *n*-pentane and its isomers, any of the hexyl radicals derived from *n*-hexane and its isomers, etc.; or an alkoxy radical composed of any one of the foregoing alkyl radicals and an oxygen atom.

More specifically, they include N-phenyl alkyl carbamates of the formula (II) in which $R_1$ is an alkyl radical as defined above and $R_2$ is a hydrogen atom; N-halophenyl alkyl carbamates of the general formula (II) in which $R_1$ is an alkyl radical as defined above and $R_2$ is a halogen atom as defined above; N-alkylphenyl alkyl carbamates of the general formula (II) in which $R_1$ and $R_2$ are alkyl radicals as defined above; N-alkoxyphenyl alkyl carbamates of the general formula (II) in which $R_1$ is an alkyl radical as defined above and $R_2$ is an alkoxy radical as defined above; N-phenyl cyclopentyl or cyclohexyl carbamate of the general formula (II) in which $R_1$ is a cyclopentyl or cyclohexyl radical and $R_2$ is a hydrogen atom; N-halophenyl cyclopentyl or cyclohexyl carbamates of the general formula (II) in which $R_1$ is a cyclopentyl or cyclo-hexyl radical and $R_2$ is a halogen atom as defined above; N-alkylphenyl cyclopentyl or cyclohexyl carbamates of the general formula (II) in which $R_1$ is a cyclopentyl or cyclohexyl radical and $R_2$ is an alkyl radical as defined above; and N-alkoxyphenyl cyclopentyl or cyclohexyl carbamates of the general formula (II) in which $R_1$ is a cyclopentyl or cyclohexyl radical and $R_2$ is an alkoxy radical as defined above.

The preferred N-phenyl carbamic acid ester are N-phenyl methyl carbamate, N-phenyl ethyl carbamate, N-phenyl *n*-propyl carbamate, N-phenyl isopropyl carbamate, N-phenyl *n*-butyl carbamate, N-phenyl *n*-butyl carbamate, N-phenyl *sec*-butyl carbamate, N-phenyl isobutyl carbamate, N-phenyl *tert*-butyl carbamate, N-phenyl pentyl carbamate, N-phenyl hexyl carbamate, N-*o*-chlorophenyl methyl carbamate, N-*o*-chlorophenyl ethyl carbamate, N-*o*-chlorophenyl isopropyl carbamate, N-*o*-chlorophenyl isobutyl carbamate, N-*o*-methylphenyl methyl carbamate, N-*o*-methylphenyl ethyl carbamate, N-phenyl cyclohexyl carbamate, N-*o*-chlorophenyl cyclohexyl carbamate, N-*o*-methylphenyl cyclohexyl carbamate, N-*m*-methoxyphenyl methyl carbamate, N-phenyl cyclopentyl carbamate, and the like.

The formaldehyde-producing substance used in the process of the present invention is a compound selected from the group consisting of paraformaldehyde, trioxane, methylal and other formals. Usually, an aqueous formaldehyde solution or paraformaldehyde is used.

The ferric halide compound used as the catalyst in the process of the present invention is a chloride or bromide of iron, and specific examples thereof include ferric chloride, ferric bromide and compounds containing them, as well as mixtures thereof. They may be used in the form of anhydrous salts, hydrous salts, aqueous solutions or organic solvent solutions, and the accompanying unnecessary water or organic solvent can be removed, either before or during the reaction, from the system according to any suitable technique such as distillation or the like. Mixtures consisting of ferric chloride or ferric bromide and ferrous chloride, ferrous bromide, iron hydroxide, iron oxide, iron oxychloride or the like can also be used. In addition, compounds which can be converted into ferric halide compounds in the reaction system also come within the category of the catalysts useful in the process of the present invention. Among them, ferric chloride compounds are particularly preferred.

The solvent used in the process of the present invention preferably has the properties of being stable under the reaction conditions, being substantially inert to formaldehyde, and dissolving the N-phenyl carbamic acid ester used as a starting material and the product consisting of polymethylene polyphenyl polycarbamates. Specific examples of the solvent include halogenated or nitrated aliphatic hydrocarbons such as chloroform, dichloroethane, nitromethane, etc.; aromatic hydrocarbons and halogenated or nitrated derivatives thereof, such as benzene, chlorobenzene, dichlorobenzene, nitrobenzene, etc.; ethers such as diethyl ether, diisopropyl ether, tetrahydrofuran, tetrahydropyran, dioxan, etc.; and alcohols such as ethyl alcohol, isopropyl alcohol, butyl alcohol, etc. Among them, solvents which can form an azeotrope with water are preferred, and benzene and dichloroethane are particularly preferred.

Generally speaking, the process of the present invention may be performed by mixing a solvent, an N-phenyl carbamic acid ester, formaldehyde and a catalyst, heating the resulting reaction mixture to a predetermined temperature, and stirring it in a substantially homogeneous system while, if necessary, removing the water introduced by the starting materials and the catalyst and the water produced by the reaction from the system. Alternatively, the process of the present invention may also be performed in a semibatch operation in which a solution of formaldehyde is added dropwise to a solution of an N-phenyl carbamic acid ester. In this case, the catalyst may be present in either of the solutions or may be added separately. Furthermore, the process of the present invention may also be performed in a continuous operation in which a solution containing the starting materials and/or the catalyst in an appropriate proportion is continuously fed to a reactor and continuously withdrawn therefrom after a predetermined residence time.

The N-phenyl carbamic acid ester and the formaldehyde or formaldehyde-producing substance are usually used in such amounts as to provide from 0.01 to 2 moles, preferably from 0.1 to 1 mole, of formaldehyde per mole of the N-phenyl carbamic acid ester. Although the amount of formaldehyde may be less than 0.01 mole, an undesirably large amount of the N-phenyl carbamic acid ester remains unreacted in such a case. If it is greater than 2 moles, the resulting composition contains an undesirably high proportion of polynuclear compounds.

The ferric halide compound, which acts as the catalyst, must be used in an amount of from 0.01 to 10 moles per mole of the N-phenyl carbamic acid ester and not less than 0.1% by weight based on the total amount of the reaction mixture. If the amount of ferric halide compound used is below these limits, the reaction becomes too slow for industrial production.

The amount of solvent used depends on the type of solvent used and its power to dissolve the starting materials and the product. However, the solvent is usually used in an amount of from 0.2 to 10 parts by weight per part by weight of the N-phenyl carbamic acid ester.

The reaction temperature may range from 40 to 180°C, the preferred range being from 60 to 140°C. If the reaction temperature is lower than 40°C, the reaction becomes too slow, while if it is higher than 180°C, undesirable side reactions such as hydrolysis and the like occur to a considerable degree.

Although the reaction may be carried out at atmospheric pressure, subatmospheric pressures somewhat lower than it are often preferred in order to accelerate the removal of water from the system. These subatmospheric pressures may range from 100 to 760 mmHg. The reaction can also be carried out at superatmospheric pressures.

The reaction time depends on the types and amounts of starting materials and catalyst used, the type of operation, reaction conditions and the like, so that no definite limitation is placed thereon. In the case of batch operations, however, it usually ranges from 0.1 to 10 hours.

In the process of the present invention, it is necessary to carry out the reaction in a substantially homogeneous system. Depending on the types of starting materials, catalyst and solvent used, therefore, it may be preferable to carry out the reaction while removing the water introduced and the water produced by the reaction from the system as much as possible. Generally speaking, the removal of water from the system can preferably be accomplished by azeotropic distillation with the solvent used. This operation is desirably performed continuously, though it may be performed intermittently. If the reaction is carried out in the presence of a large amount of water and hence in a heterogeneous system, the formation of by-products such as N-benzyl compounds and anilinomethane compounds may be so much increased that the degree of selectivity for the desired product and the yield thereof are decreased.

According to the process of the present invention, the ratio of binuclear compounds to trinuclear or higher polynuclear compounds present in the product can be changed by properly controlling the molar ratio of N-phenyl carbamic acid ester to formaldehyde. For example, if the molar ratio of formaldehyde to N-phenyl carbamic acid ester is in the range of 0.01 to 0.4, a product containing a relatively high proportion of binuclear compounds (that is, a composition consisting of 100—60% of binuclear compounds and 0—40% of trinuclear or higher polynuclear compounds) is obtained. On the other hand, if the molar ratio is greater than 0.4, a product containing a relatively high proportion of trinuclear or higher polynuclear compounds (that is, a composition consisting of 70% or less of binuclear compounds and 30% or more of trinuclear or higher polynuclear compounds) is obtained. The former product is suitable for the manufacture of polyisocyanates for use in applications such as synthetic leather and the like, while the latter is suitable for the manufacture of polymeric isocyanates for use in rigid polyurethanes.

According to the process of the present invention, the formation of undesirable by-products such as N-benzyl compounds and anilinomethane compounds is markedly decreased as compared with well-known prior art processes using a dilute aqueous solution of an acid as the catalyst. Usually, such by-products are hardly detectable or found in an amount of at most 4% by weight. It is generally unnecessary, therefore, to subject the product to an additional step of converting the N-benzyl compounds secondarily (as employed in the aforementioned U.S. Patent No. 4,146,727).

After completion of the reaction carried out according to the above-described procedure, water or an aqueous solution of a hydrohalogenic acid is added to the reaction mixture and a catalytic component containing the ferric halide compound is separated in the form of an aqueous phase to leave an organic phase containing the desired product. The desired product can be obtained by removing the solvent and unreacted N-phenyl carbamic acid ester from the organic phase according to any well-known technique such as distillation, extraction or the like. If necessary, this product may further be subjected to purifying operations such as distillation, recrystallization, extraction and the like to remove any impurities and thereby obtain a product of higher quality. Where it is desired to prepare the corresponding polyisocyanates by thermal decomposition, the product containing the solvent and unreacted N-phenyl carbamic acid ester can be directly used as the raw material for thermal decomposition, though it depends on the method of thermal decomposition.

In a preferred embodiment of the present invention, the catalyst is recovered from the reaction mixture having passed through the above-described procedure and then used repeatedly to prepare polymethylene polyphenyl polycarbamates. Specifically, after completion of the reaction, water or an aqueous solution of a hydrohalogenic acid is added to the reaction mixture containing the desired product. Thus, the catalytic component containing the ferric halide compound is extracted in an aqueous phase and then reused.

In order to extract the ferric halide compound, it is usually preferable to use an aqueous solution of a hydrohalogenic acid. The use of an aqueous solution of a hydrohalogenic acid is advantageous in

that the recovery of the catalyst component is high, the catalytic component which has been slightly deteriorated during the reaction is effectively activated, and the excess hydrohalogenic acid, together with the catalytic component, can be efficiently utilized in a subsequent reaction.

Moreover, as the ferric halide component is recycled several times in the above-described manner, the N-benzyl compound which is usually formed in small amounts becomes hardly detectable for some unknown reason.

On analysis of the solution of the ferric halide compound recovered for repeated use, it has been found that part of the ferric ions are converted into ferrous ions. Accordingly, the coexistence of a ferrous compound in the ferric halide compound used as the catalyst causes no inconvenience to the present reaction. On the contrary, this may at least partially account for the favorable result that recovery and recycling of the catalyst eliminates the formation of N-benzyl compounds.

The water or aqueous solution which is added to the reaction mixture in order to recover the ferric halide compound therefrom is preferably used in an amount of at least 1.0 times the weight of the ferric halide compound and from 0.1 to 10 times the weight of the reaction mixture. Where an aqueous solution of a hydrohalogenic acid is added for this purpose, it is desirably used in such an amount as to provide from 0.001 to 10 moles, preferably from 0.01 to 3.0 moles, of the hydrohalogenic acid per mole of the ferric halide compound. If the amount of water or aqueous solution used is outside these ranges, recovery of the catalyst and separation of the aqueous phase from the organic phase cannot be accomplished with success.

The catalyst-containing aqueous phase obtained by adding water or an aqueous solution of a hydrohalogenic acid to the reaction mixture and separating the resulting aqueous phase from the organic phase is reused as a catalyst solution after its concentration is adjusted, if necessary, according to any suitable method. This can be done, for example, by concentrating the aqueous phase or by adding a suitable solvent thereto and subjecting the resulting mixture to azeotropic distillation. The concentration adjustment of the aqueous phase reused as a catalyst solution may be made either by treating the separated and recovered aqueous phase separately or by adding it to a subsequent reaction system and removing water therefrom during the course of the reaction.

Thus, the greater part of the catalyst used in the process of the present invention can be repeatedly used without any particular limit, if only the slight losses caused by manipulation and/or mixing in the product are made up according to the need. In cases where the catalyst is used repeatedly, the desired product can be obtained by removing the solvent and unreacted N-phenyl carbamic acid ester from the organic phase left after the aqueous phase has been separated as described above.

As can be seen from the above description, the process of the present invention has the following advantages over well-known prior art processes and, therefore, is of great utility in industrial applications.

(1) An easily obtainable, inexpensive and highly active catalyst is used.

(2) The reaction may be carried out according to a simple procedure and under mild conditions.

(3) A higher reaction rate can be achieved.

(4) Only a little or virtually no formation of undesirable by-products such as N-benzyl compounds and anilinomethane compounds is noted and, therefore, the desired product is obtained with high selectivity and in good yield.

(5) Compositions consisting of polymethylene polyphenyl polycarbamates in a proportion suitable for the manufacture of industrially useful polyisocyanates can be obtained.

(6) The used catalyst may be readily recovered and reused according to a simple procedure.

The process of the present invention is further illustrated by the following examples. In these examples, the product was analyzed by liquid chromatography using naphthalene as an internal standard.

### Example 1

Into a 500-ml round-bottomed flask fitted with a thermometer, a stirrer, a dropping funnel, and a reflux condenser having a separator were charged 90 g of N-phenyl ethyl carbamate and 180 g of benzene. While the flask was being heated at 80°C to reflux the benzene, 44.3 g of a 40% aqueous solution of ferric chloride was added dropwise with water being removed azeotropically from the system. Subsequently, 22.05 g of a 37% aqueous solution of formaldehyde was added dropwise over a period of 2 hours and, thereafter, the resulting reaction mixture was matured by allowing it to stand for 3 hours. After completion of the reaction, 100 g of a 2% aqueous solution of hydrochloric acid was added to and stirred with the reaction mixture, and 117 g of an aqueous phase was then separated by means of a separatory funnel. After the benzene layer was washed with water, the benzene was distilled off to obtain 91.1 g of a product. On analysis by liquid chromatography, the product was found to contain 14.7 g of unreacted N-phenyl ethyl carbamate, 40.9 g of binuclear compounds, 18.4 g of trinuclear compounds, 16.2 g of tetranuclear or higher polynuclear compounds, and 0.9 g of [(ethoxycarbonyl)phenylaminomethyl)]phenyl carbamic acid ethyl ester known as an N-benzyl compound. These results indicate that the degree of conversion of the N-phenyl ethyl carbamate was 83.7%, the yield (or the degree selectivity) based on the amount of N-phenyl ethyl carbamate consumed was 52.8% for binuclear compounds, 23.5% for trinuclear compounds, and 20.6% for tetranuclear or higher

polynuclear compounds, and the yield of the N-benzyl compound was as low as 1.2%.

On analysis of the separated aqueous phase for iron, its recovery was found to be 99%.

Example 2

In order to make up for the storage, an appropriate amount of a 40% aqueous solution of ferric chloride was added to the aqueous phase recovered in Example 1. Using it as a catalyst solution, the same reaction as described in Example 1 was carried out again. In the manner, the recovered aqueous solution of ferric chloride was recycled in a total of 8 repeated runs. The results thus obtained are shown in Table 1. As can be seen from this table, the aqueous solution of ferric chloride could be reused without any reduction in activity, and the N-benzyl compound which had initially been formed in a small but appreciable amount was hardly detectable in the third and further repeated turns.

TABLE 1

Charged Materials for Each Run: 90 g of N-phenyl ethyl carbamate, 22.05 g of a 37% aqueous solution of formaldehyde, and 180 g of benzene.

Reaction Conditions: 80°C, 5 hours.

| Number of Repeated Run | Recovered Aqueous Ferric Chloride Solution | | Amount of 40% Aqueous Ferric Chloride Solution Added (g) | Yield of Product (g) | Degree of Conversion of N-Phenyl Ethyl Carbamate (%) | Yield (or Degree of Selectivity) Based on N-Phenyl Ethyl Carbamate Consumed (%) | | | |
|---|---|---|---|---|---|---|---|---|---|
| | Amount (g) | Recovery (%) | | | | Binuclear Compounds | Trinuclear Compounds | Tetranuclear or Higher Polynuclear Compounds | N-Benzyl Compound |
| Initial run | 117 | 99 | 44.3 | 91.1 | 83.7 | 52.3 | 23.5 | 20.6 | 1.2 |
| 1 | 115 | 85 | 0.4 | 94.4 | 84.5 | 52.6 | 23.9 | 19.2 | 0.5 |
| 2 | 117 | 99 | 0.6 | 92.8 | 81.9 | 51.5 | 25.3 | 21.6 | 0.4 |
| 3 | 118 | 94 | 0.4 | 92.5 | 83.2 | 50.5 | 25.1 | 23.3 | Trace |
| 4 | 115 | 86 | 2.7 | 91.7 | 84.4 | 51.8 | 27.0 | 21.2 | Trace |
| 5 | 117 | 95 | 6.2 | 92.0 | 83.2 | 51.2 | 23.8 | 23.4 | Trace |
| 6 | 118 | 92 | 2.2 | 91.7 | 84.7 | 51.1 | 25.4 | 23.5 | 0 |
| 7 | 116 | 93 | 5.5 | 92.8 | 83.7 | 50.7 | 24.4 | 21.9 | 0 |
| 8 | 116 | 90 | 3.1 | 89.5 | 83.6 | 51.9 | 25.0 | 21.4 | 0 |

Example 3

The procedure of Example 1 was repeated except that 9.80 g of a 37% aqueous solution of formaldehyde was added dropwise over a period of 50 minutes. As a result, the degree of conversion of the N-phenyl ethyl carbamate was 46.2%, and the degree of selectivity based on the amount of N-phenyl ethyl carbamate consumed was 76.5% for binuclear compounds, 16.5% for trinuclear compounds, and 4.0% for tetranuclear or higher polynuclear compounds. Thus, there was obtained a product containing a higher proportion of binuclear compounds than that of Example 1.

Example 4

Into a 100-ml round-bottomed flask fitted with a thermometer, a stirrer, a dropping funnel, and a reflux condenser having a separator were charged with 20 g of N-phenyl ethyl carbamate, 40 g of benzene, 16.2 g of ferric chloride hexahydrate, and 1.8 g of paraformaldehyde. Then, reaction was carried out at 80°C for 5 hours with water being azeotropically removed from the system. As a result, the degree of conversion of the N-phenyl ethyl carbamate was 85.1%, and the degree of selectivity based on the amount of N-phenyl ethyl carbamate consumed was 47.5% for binuclear compounds, 27.5% for trinuclear compounds, 25.0% for tetranuclear or higher polynuclear compounds, and a trace for the N-benzyl compound.

Example 5

The procedure of Example 4 was repeated except that 9.7 g of anhydrous ferric chloride was used in place of the ferric chloride hexahydrate. As a result, the degree of conversion of the N-phenyl ethyl carbamate was 76%, and the degree of selectivity based on the amount of N-phenyl ethyl carbamate consumed was 58.0% for binuclear compounds, 27.0% for trinuclear compounds, 10.5% for tetranuclear or higher polynuclear compounds, and 0.8% for the N-benzyl compound.

Example 6

The procedure of Example 5 was repeated except that 1,2-dichloroethane was used in place of the benzene and the removal of water from the system during the course of the reaction was omitted. As a result, the degree of conversion of the N-phenyl ethyl carbamate was 83%, and the degree of selectivity was 53.8% for binuclear compounds, 24% for trinuclear compounds, 21.1% for tetranuclear or higher polynuclear compounds, and 1.0% for the N-benzyl compound.

Comparative Example 1

In the same manner as described in Example 4, the reaction of N-phenyl ethyl carbamate with formaldehyde was carried out at 98°C for 5 hours. In this comparative example, however, no benzene was used, 62 g of a 7% aqueous solution of hydrochloric acid was used in place of the ferric chloride hexahydrate, and the removal of water from the system during the course of the reaction was omitted. As a result, the degree of conversion of the N-phenyl ethyl carbamate was 48.0%, and the degree of selectivity was 60.0% for binuclear compounds, 5.0% for trinuclear compounds, 2.5% for tetranuclear or higher polynuclear compounds, 2.5% for the N-benzyl compound, and 5.0% for the anilinomethane compound, namely bis(N-carboethoxyanilino)methane.

Comparative Examples 2—6

The procedure of Example 4 was repeated except that various catalysts were used in place of the ferric chloride hexahydrate. The catalysts used and the results thus obtained are shown in Table 2.

TABLE 2

Charged Materials: 20 g of N-phenyl ethyl carbamate, 1.8 g of paraformaldehyde, and 40 g of benzene.
Reaction Conditions: 80°C, 5 hours.

| Comparative Example | Catalyst | | Degree of Conversion of N-Phenyl Ethyl Carbamate (%) | Degree of Selectivity Based on N-Phenyl Ethyl Carbamate Consumed (%) | | | | |
|---|---|---|---|---|---|---|---|---|
| | Type | Amount (g) | | Binuclear Compounds | Trinuclear Compounds | Tetranuclear or Higher Polynuclear Compounds | N-Benzyl Compound | Anilinomethane Compound |
| 2 | Zinc chloride | 8.2 | 50 | 23.2 | 13.8 | 5.0 | 32.3 | 20.6 |
| 3 | Aluminum chloride hexahydrate | 14.5 | 14 | Trace | 0 | 0 | Trace | 69.0 |
| 4 | Chromium chloride trihydrate | 16.0 | 13 | Trace | 0 | 0 | 15.4 | 76.1 |
| 5 | Nickel chloride hexahydrate | 14.3 | 0 | 0 | 0 | 0 | 0 | 0 |
| 6 | Anhydrous aluminum chloride | 8.0 | 53 | 71.0 | 7.9 | 2.5 | 11.3 | Trace |

Examples 7—10

The procedure of Example 4 was repeated except that several N-phenyl carbamic acid esters were used as a starting material and several ferric halide compounds were used as the catalyst in different amounts. The starting materials and catalysts used and the results thus obtained are shown in Table 3.

TABLE 3

Charged Materials: 0.12 mole of an N-phenyl carbamic acid ester, 0.024 mole of a catalyst, 1.8 g of paraformaldehyde, and 40 g of benzene.

Reaction Conditions: 80°C, 5 hours.

| Example | N-Phenyl Carbamic Acid Ester | Catalyst | | Degree of Conversion of N-Phenyl Carbamic Acid Ester (%) | Degree of Selectivity Based on N-Phenyl Carbamic Acid Ester Consumed (%) | | | | |
|---|---|---|---|---|---|---|---|---|---|
| | | Type | Amount (g) | | Binuclear Compounds | Trinuclear Compounds | Tetranuclear or Higher Polynuclear Compounds | N-Benzyl Compound | Anilinomethane Compound |
| 7 | Ethyl ester | Ferric chloride hexahydrate | 6.48 | 77.3 | 55.2 | 23.8 | 18.3 | 1.8 | 0 |
| 8 | Methyl ester | Ferric chloride hexahydrate | 6.48 | 78.2 | 51.2 | 25.4 | 22.8 | 0.4 | 0 |
| 9 | Isopropyl ester | Ferric chloride hexahydrate | 6.48 | 79.2 | 54.0 | 18.7 | 16.4 | 4.0 | Trace |
| 10 | Ethyl ester | Ferric bromide | 7.09 | 72.2 | 55.4 | 16.8 | 8.9 | 4.0 | Trace |

## Claims

1. A process for preparing a polymethylene polyphenyl polycarbamate of the general formula

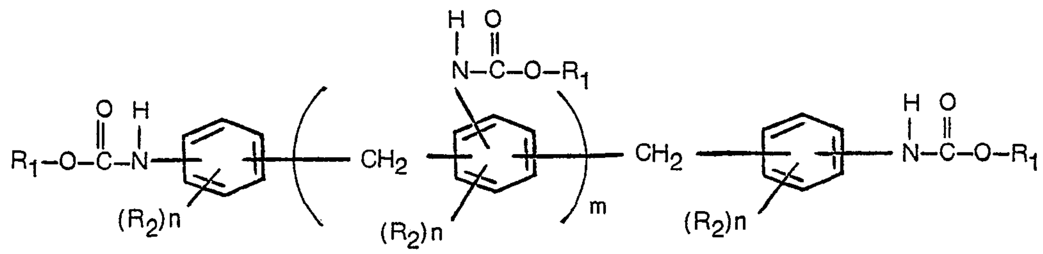

(I)

where $R_1$ is a lower alkyl radical of from 1 to 6 carbon atoms or a cycloalkyl radical, $R_2$ is a hydrogen atom, a halogen atom, a lower alkyl radical from 1 to 6 carbon atoms, or a lower alkoxy radical of from 1 to 6 carbon atoms, n is a positive integer of from 1 to 3, and m is zero or a positive integer of from 1 to 5, by reacting an N-phenyl carbamic acid ester of the general formula

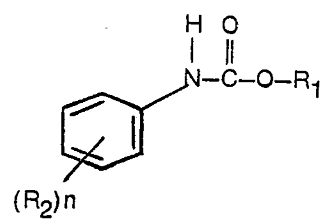

(II)

where $R_1$, $R_2$ and n have the same meanings as described above, with formaldehyde or a formaldehyde-producing substance, characterised by

(a) using a ferric halide compound as the catalyst in an amount of from 0.01 to 10 moles per mole of the N-phenyl carbamic acid ester and not less than 0.1% by weight based on the total amount of the reaction mixture, and

(b) carrying out the reaction in the presence of a solvent and in a substantially homogeneous system.

2. The process according to claim 1 wherein $R_1$ is a lower alkyl radical selected from the group consisting of methyl, ethyl and isopropyl radicals and $R_2$ is a hydrogen atom.

3. The process according to claim 1 wherein the formaldehyde-producing substance is a member selected from the group consisting of paraformaldehyde, trioxane, methylal and ethylal.

4. The process according to claim 1 wherein the ferric halide compound is ferric chloride or ferric bromide.

5. The process according to claim 1 wherein the solvent is benzene.

6. The process according to claim 1 wherein the solvent is dichloroethane.

7. The process according to claim 1 wherein the N-phenyl carbamic acid ester and the formaldehyde or formaldehyde-producing substance are used in such amounts as to provide from 0.01 to 2 moles of formaldehyde per mole of the N-phenyl carbamic acid ester.

8. The process according to claim 1 wherein the reaction temperature is in the range of from 60 to 140°C.

9. The process according to claim 1 wherein $R_1$ is a methyl or ethyl radical, $R_2$ is a hydrogen atom, the formaldehyde-producing substance is a member selected from the group consisting of paraformaldehyde, trioxane, methylal and ethylal, the ferric halide compound is ferric chloride, and the solvent is benzene or dichloroethane.

10. The process according to any one of claims 1 to 9 wherein, after completion of the reaction, water or an aqueous solution of a hydrohalogenic acid is added to the reaction mixture and the resulting aqueous phase containing the ferric halide compound is separated and recovered to reuse the catalyst.

11. The process according to claim 10 wherein the hydrohalogenic acid is hydrochloric acid.

12. The process according to claim 10 wherein the water is used in an amount of at least 10 times the weight of the ferric halide compound and from 0.1 to 10 times the weight of the reaction mixture, or the aqueous solution of the hydrohalogenic acid is used in such an amount as to provide from 0.01 to 3 moles of the hydrohalogenic acid per mole of the ferric halide compound.

**Patentansprüche**

1. Verfahren zur Herstellung eines Polymethylen-polyphenyl-polycarbamats der allgemeinen Formel

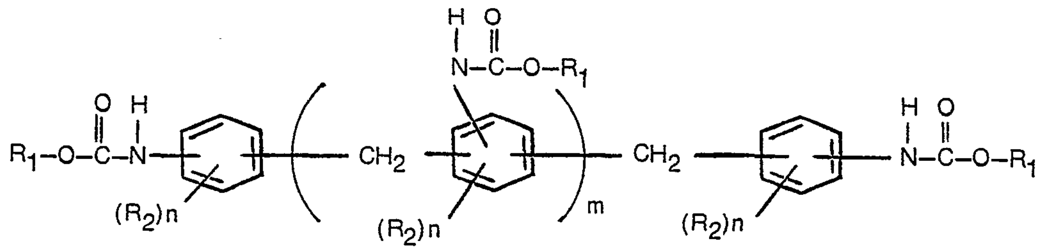

(I)

worin $R_1$ einen Neidrigalkylrest mit 1 bis 6 Kohlenstoffatomen oder einen Cycloalkylrest darstellt, $R_2$ ein Wasserstoffatom, ein Halogenatom, einen Niedrigalkylrest mit 1 bis 6 Kohlenstoffatom oder einen Niedrigalkoxyrest mit 1 bis 6 Kohlenstoffatom bedeutet, n eine positive ganze Zahl von 1 bis 3 ist und m Null oder eine positive ganze Zahl von 1 bis 5 bedeutet, durch Umsetzung eines n-Phenylcarbamidsäureesters der allgemeinen Formel

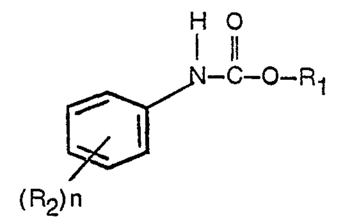

(II)

worin $R_1$, $R_2$ und n die vorstehend angegebene Bedeutung besitzen, mit Formaldehyd oder einer Formaldehyd-bildenden Substanz, dadurch gekennzeichnet, daß man

(a) eine Ferrihalogenidverbindung als Katalysator in einer Menger von 0,01 bis 10 Mol je Mol des N-Phenylcarbamidsäureesters und von nicht weniger als 0,1 Gewichtsprozent, bezogen auf die Gesamtmenge der Reaktionsmischung, verwendent und

(b) die Umsetzung in Gegenwart eines Lösungsmittels und in einem im wesentlichen homogenen System durchführt.

2. Verfahren gemäß Anspruch 1, dadurch gekennzeichnet, daß $R_1$ eine Niedrigalkylrest, ausgewählt unter den Methyl-, Äthyl- und Isopropylresten, ist und $R_2$ ein Wasserstoffatom bedeutet.

3. Verfahren gemäß Anspruch 1, dadurch gekennzeichnet, daß die Aldehyd-bildende Substanze ausgewählt wird unter para-Formaldehyd, Trioxan, Methylal und Äthylal.

4. Verfahren gemäß Anspruch 1, dadurch gekennzeichnet, daß die Ferrihalogendverbindung Ferrichlorid oder Ferribromid ist.

5. Verfahren gemäß Anspruch 1, dadurch gekennzeichnet, daß das Lösungsmittel Benzol ist.

6. Verfahren gemäß Anspruch 1, dadurch gekennzeichnet, daß das Lösungsmittel Dichloräthan ist.

7. Verfahren gemäß Anspruch 1, dadurch gekennzeichnet, daß der N-Phenylcarbamidsäureester und der Formaldehyd oder die Formaldehyd-bildende Substanz in derartigen Mengen eingesetzt werden, daß sich 0,01 bis 2 Mol Formaldehyd je Mol N-Phenylcarbamidsäureester ergeben.

8. Verfahren gemäß Anspruch 1, dadurch gekennzeichnet, daß die Reaktionstemperatur im Bereich von 60 bis 140°C liegt.

9. Verfahren gemäß Anspruch 1, dadurch gekennzeichnet, daß $R_1$ einen Methyl- oder Äthylrest bedeutet, $R_2$ ein Wasserstoffatom bedeutet, die Formaldehyd-bildende Substanz ausgewählt wird unter para-Formaldehyd, Trioxan, Methylal und Äthylal, die Ferrihalogenidverbindung Ferrichlorid ist und das Lösungsmittel Benzol oder Dichloräthan ist.

10. Verfahren gemäß einem der Ansprüche 1 bis 9, dadurch gekennzeichnet, daß nach Beendigung der Umsetzung Wasser oder eine wäßrige Lösung einer Halogenwasserstoffsäure zu der Reaktionsmischung zugegeben wird und die die Ferrihalogenidverbindung enthaltende wäßrige Phase abgetrennt und für die Wiederverwendung des Katalysators gewonnen wird.

11. Verfahren gemäß Anspruch 10, dadurch gekennzeichnet, daß die Halogenwasserstoffsäure Chlorwasserstoffsäure ist.

12. Verfahren gemäß Anspruch 10, dadurch gekennzeichnet, daß das Wasser in einer Menge von zumindest dem 10-fachen des Gewichts der Ferrihalogenidverbindung und von 0,1- bis 10-fachen des Gewichts der Reaktionsmischung verwendet wird, oder die wäßrige Lösung der Halogenwasserstoffsäure in einer derartigen Menge verwendet wird, daß sich 0,01 bis 3 Mol Halogenwasserstoffsäure je Mol Ferrihalogenidverbindung ergeben.

**Revendications**

1. Procédé pour préparer un polyméthylène polyphényl polycarbamate représenté par la formula générale:

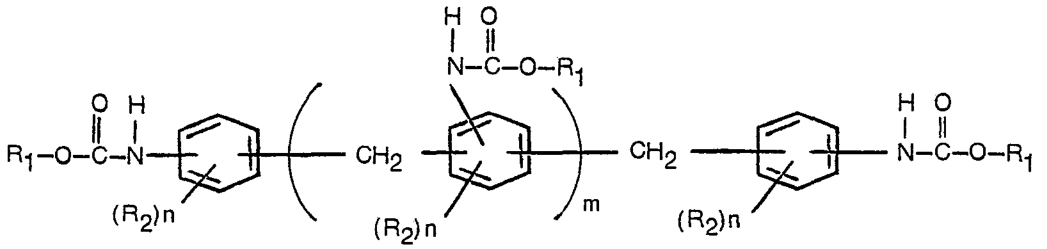

(I)

dans laquelle $R_1$ est un radical alcoyle inférieur de 1 à 6 atomes de carbone ou un radical cycloalcoyle, $R_2$ est un atome d'hydrogène, un atome d'halogène, un radical alcoyle inférieur de 1 à 6 atomes de carbone, ou un radical alcoxy inférieur de 1 à 6 atomes de carbone, n est un entier positif de 1 à 3, et m est zéro ou un entier positif de 1 à 5, par la réaction d'un ester d'acide N-phényl carbamique représenté par la formule générale:

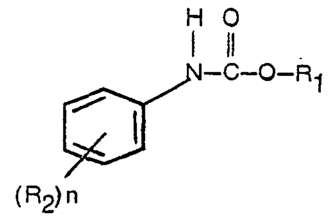

(II)

dans laquelle $R_1$, $R_2$ et n sont tels que définis ci-dessus, avec du formaldéhyde ou une substance produisant du formaldéhyde, caractérisé in ce que:

(a) on utilise un composé d'halogénure ferrique comme catalyseur en une quantité de 0,01 à 10 moles par mole d'ester d'acide N-phényl carbamique et pas moins de 0,1% en poids par rapport à la quantité totale du mélange de réaction, et

(b) on effectue la réaction en présence d'un solvant et dans un système pratiquement homogène.

2. Procédé selon la revendication 1, caractérisé en ce que $R_1$ est un radical alcoyle inférieur choisi dans le groupe formé par les radicaux méthyle, éthyle et isopropyle, et $R_2$ est un atome d'hydrogène.

3. Procédé selon la revendication 1, caractérisé en ce que la substance produisant du formaldéhyde est choisi dans le groupe consistant en paraformaldéhyde, trioxanne, méthylal et éthylal.

4. Procédé selon la revendication 1, caractérisé en ce que le composé d'halogénure ferrique est du chlorure ferrique ou du bromure ferrique.

5. Procédé selon la revendication 1, caractérisé en ce que le solvant est du benzène.

6. Procédé selon la revendication 1, caractérisé en ce que le solvant est du dichloroéthane.

7. Procédé selon la revendication 1, caractérisé en ce que l'ester d'acide N-phényl carbamique et le formaldéhyde ou la substance produisant du formaldéhyde sont utilisés en des quantités telles que cela fournit de 0,01 à 2 moles de formaldéhyde par mole d'ester d'acide N-phényl carbamique.

8. Procédé selon la revendication 1, caractérisé en ce que la température de réaction est dans la gamme de 60 à 140°C.

9. Procédé selon la revendication 1, caractérisé en ce que $R_1$ est un radical méthyle ou éthyle, $R_2$ est un atome d'hydrogène, la substance produisant du formaldéhyde est choisie dans le groupe consistant en paraformaldéhyde, trioxanne, méthylal et éthylal, le composé d'halogénure ferrique est du chlorure ferrique, et le solvant est du benzène ou du dichloroéthane.

10. Procédé selon l'une des revendications 1 à 9, caractérisé en ce que, après achèvement de la réaction, de l'eau ou une solution aqueuse d'un acide halohydrique est ajoutée au mélange de réaction et que la phase aqueuse résultante contenant le composé d'halogénure ferrique est séparée et récupérée pour réutiliser le catalyseur.

11. Procédé selon la revendication 10, caractérisé en ce que l'acide halohydrique est de l'acide chlorhydrique.

12. Procédé selon la revendication 10, caractérisé en ce que l'eau est utilisée en une quantité d'au moins 10 fois le poids du composé d'halogénure ferrique et de 0,1 à 10 fois le poids du mélange de réaction, ou que la solution aqueuse d'acide halohydrique est utilisée en une quantité telle qu'elle fournisse de 0,01 à 3 moles d'acide halohydrique par mole de composé d'halogénure ferrique.